(19) Europäisches Patentamt
European Patent Office
Office européen des brevets

(11) **EP 1 602 367 A1**

(12) **EUROPEAN PATENT APPLICATION**

(43) Date of publication:
07.12.2005 Bulletin 2005/49

(51) Int Cl.⁷: **A61K 9/70**, A61K 31/40

(21) Application number: 05076267.3

(22) Date of filing: 31.05.2005

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **01.06.2004 US 575848 P**

(71) Applicant: **Axonyx, Inc.**
**New York, NY 10018 (US)**

(72) Inventor: **Bruinsma, Gosse B.**
**2311 XD Leiden (NL)**

(74) Representative:
**Winckels, Johannes Hubertus F. et al**
**Vereenigde,**
**Johan de Wittlaan 7**
**2517 JR Den Haag (NL)**

(54) **Transdermal delivery system for treatment of cognitive disorders**

(57)    An improved transdermal delivery composition is provided containing a low molecular weight fatty acid, and a long chain fatty acid or a long chain fatty acid lower alkyl ester, wherein the long chain is between about 1 to about 21 carbons long and preferably, contains at least one cis-olefinic bond. Preferred long chain compo- nents are oleic acid and ethyl oleate while the preferred low molecular weight fatty acid is propionic acid. The product may be used for treating a cognitive disorder by administering phenserine through a subject's skin.

EP 1 602 367 A1

## Description

### CROSS-REFERENCE TO RELATED APPLICATIONS

This application claims the benefit of U.S. Provisional Application No. 60/575,848, filed June 1, 2004, the entirety of which is incorporated by reference.

### TECHNICAL FIELD

This invention relates to biotechnology, and more particularly to methods of treating cognitive disorders using the cholinesterase inhibitor phenserine.

### BACKGROUND

The delivery of drugs by the transdermal route is an area of increasing interest and offers the advantage of allowing a prolonged, steady input of drug into the blood. However, the excellent barrier properties of the skin impose a significant obstacle to the delivery of the required dosage.

Transdermal delivery avoids hepatic "first pass" metabolism, the pain associated with injections, and may increase dosage compliance.

Transdermal drug delivery devices typically involve a carrier (such as a liquid, gel, or solid matrix, or a pressure sensitive adhesive) into which the drug to be delivered is incorporated. The drug-containing carrier is then placed on the skin and the drug, along with any adjuvants and excipients, is delivered to the skin.

Typically the portions of the carrier that are not in contact with the subject's skin are covered by a backing. The backing serves to protect the carrier (and the components contained in the carrier, including the drug) from the environment and prevents loss of the ingredients of the drug delivery device to the environment. Because hydration of the stratum corneum is known to enhance transport of certain drugs across the skin, it is sometimes desirable that the backing have a relatively low moisture vapor transmission rate in order to retain moisture at the site covered by the drug delivery device. In order to maintain the health of the covered skin during long term wear (e.g., for periods in excess of a day) by allowing the skin to breath, it is also desirable that the backing have relatively high permeability to oxygen. Further, as the backing is in contact with the components of the carrier, including the drug and any adjuvants and excipients, it is important that the backing be stable to such components in order that the backing retains its structural integrity, tensile strength, and conformability to the skin. It is also desirable that the backing not absorb drug or other excipients from the carrier. In connection with the preparation of certain reservoir-type transdermal drug delivery devices, it is also desirable for the backing to be heat sealable at a relatively low temperature to itself and to a variety of other polymeric substrates. Backing materials that have found use in transdermal drug delivery devices include metal foils, metalized plastic films, and single layered and multilayered polymeric films (*see* U.S. Patent 5,264,219).

### SUMMARY OF THE INVENTION

In one aspect, the present invention relates to a method for the delivery of drugs to improve the effectiveness of the drug in the treatment of cognitive disorders. The invention further relates to a method of administering a compound comprising phenserine, wherein the drug may be administered in a transdermal patch.

In an exemplary embodiment, the transdermal patch includes a backing layer, which may optionally be composed of a pigmented polyester film, a drug reservoir, a microporous membrane that controls the rate of delivery of a phenserine compound from the system to the skin surface, and an adhesive formulation to attach the delivery system to a subject. Optionally, the adhesive formulation may include the phenserine compound, thus providing a more immediate bolus of phenserine upon application of the patch to a subject.

In an exemplary embodiment, the transdermal patch includes a $C_2$ to $C_4$ fatty acid in combination with oleic acid (or a $C_2$ to $C_4$ alkyl ester of oleic acid) as the vehicle for the phenserine compound.

The invention further relates to a method for treating a cognitive disorder comprising administering to a subject a first dosage of a compound comprising phenserine for a period of time, thereby allowing the subject to adapt to the first dosage; and administering a second dosage of a compound comprising phenserine to the subject, wherein the second dosage is greater than the first dosage, thereby treating the cognitive disorder. The dosages of this embodiment may be prepared in at least two different transdermal patches and may include transdermal patches of each appropriate dosage labeled in a convenient manner. Optionally, one or more additional dosages of a compound comprising phenserine may be administered for a period of time, thereby adapting the subject to the second or later dosage level; and then administering an increased dosage of a compound comprising phenserine.

The invention further relates to a kit to enhance the every day use of pharmaceutical preparations comprising phenserine for the treatment of a cognitive disorder. The invention also relates to a kit comprising a plurality of phenserine containing transdermal compositions to be administered sequentially.

The invention also relates to a method of manufacturing a pharmaceutical having beneficial properties for the treatment of cognitive disorders.

### BRIEF DESCRIPTION OF THE DRAWINGS

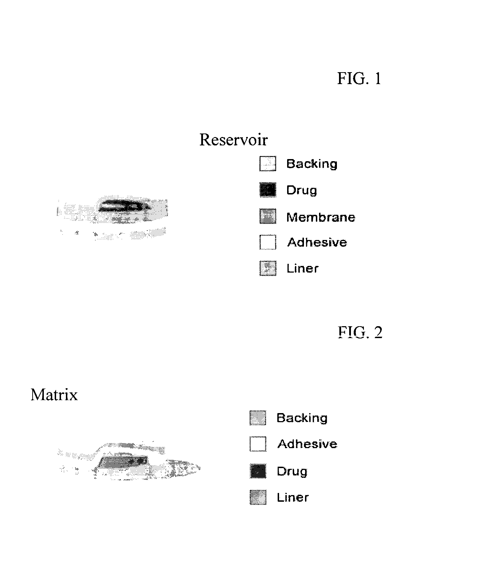

FIG. 1 illustrates a reservoir type transdermal design, which is characterized by the inclusion of a liquid compartment containing a drug solution or suspension separated from the release liner by a semipermeable membrane and adhesive. The adhesive component of the product responsible for skin adhesion may be either incorporated as a continuous layer between the membrane and the release liner or in a concentric configuration around the membrane. The liner is typically used to protect the transdermal patch during storage and shipment, and is removed prior to application to a subject. FIG. 2 illustrates a matrix design, where a semisolid matrix containing the drug solution is in direct contact with the release liner. The adhesive material is typically incorporated in an overlay and forms a concentric configuration around the semisolid matrix.

DETAILED DESCRIPTION OF THE INVENTION

The phenserine compounds of the invention may be synthesized using processes known in the art. For example, U.S. Patents 6,495,700, 5,409,948, 5,171,750, 5,378,723, and 5,998,460, and WO 03/082270 A1, all of which are hereby incorporated by reference in their entirety, describe the preparation of the phenserine compounds of the invention and enzyme inhibition assays that may be used to test compounds of the invention. The phenserine compounds of the invention are carbamates having specificity for the inhibition of acetylcholinesterase and/or inhibition of β-AAP synthesis, including, but not limited to, (-)-N-phenylcarbamoyl eseroline (which may also be referred to as (3aS)-1,3a,8-trimethyl-1,2,3,3a,8,8a-hexahydropyrrolo [2,3-b]indol-5-yl phenylcarbamate; (-)-phenserine; or phenserine).

Phenserine is a long-acting inhibitor of cholinesterase. In contrast, (3aR)-1,3a,8-trimethyl-1,2,3,3a,8,8a-hexahydropyrrolo[2,3-b]indol-5-yl phenylcarbamate (POSIPHEN™), the (+) isomer of phenserine, does not inhibit cholinesterase activity. However, (+)-phenserine, POSIPHEN™, reduces the production of beta amyloid precursor protein.

Phenserine is a relatively well tolerated drug that increases the level of acetylcholine ("ACh") by inhibition of acetylcholinesterase activity. To determine the time-dependent effects of phenserine on cholinergic function, AChE activity, brain and plasma drug levels and brain extracellular ACh concentrations were measured in rats before and after phenserine administration. Following i.v. dosing, brain drug levels were 10-fold higher than those achieved in plasma, peaked within 5 min and rapidly declined with half-lives of 8.5 and 12.6 min, respectively. In contrast, a high (> 70%) and long-lasting inhibition of AChE was achieved (half life > 8.25 h). A comparison between the time-dependent plasma AChE inhibition achieved after similar oral and i.v. doses provided an estimate of oral bioavailability of 100%. Striatal, *in vivo* microdialysis in conscious, freely-moving phenserine-treated rats demonstrated > 3-fold rise in brain ACh levels. Phenserine, thus, is rapidly absorbed and cleared from the body, but produces a long-lasting stimulation of brain cholinergic function at well tolerated doses and hence has superior properties as a drug for cognitive disorders, such as AD. Further, the selective inhibition of AChE minimizes the potential BChE side effects. Its long duration of action, coupled with its short pharmacokinetic half-life, reduces dosing frequency, decreases body drug exposure and minimizes the dependence of drug action on the individual variations of drug metabolism commonly found in the elderly.

In addition to inhibition of AChE, phenserine provides neuroprotective properties on top of the cholinergic effects, for example, reduction in β-APP production. Thus, phenserine, an acetylcholinesterase inhibitor, offers a dual mechanism of action, which should improve the outcome of subjects suffering from AD. Pharmaceutical applications of these acetylcholinesterase inhibitors may be significantly enhanced by a transdermal method of delivery.

As used herein, a "*phenserine compound*," means a compound described in U.S. Patents 5,171,750; 5,998,460; and 5,409,948, which compounds may inhibit acetylcholinesterase (e.g., phenserine), and (+)-phenserine, as well as, pharmaceutically acceptable salts or esters thereof. The term "pharmaceutically acceptable salts" includes acid addition salts, which are any non-toxic organic or inorganic acid addition salt. Illustrative inorganic acids which form suitable salts include hydrochloric, hydrobromic, sulfuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phyenoxybenzoic, and sulfonic acids such as p-toluene sulfonic acid, methanesulfonic acid and 2-hydroxyethanesulfonic acid. Preferred salts include tartrate, phosphate, and fumarate. In addition, esters, where desirable and appropriate, are included within the scope of pharmaceutically acceptable salts. The L-tartrate salt of phenserine is a water soluble compound which may beneficially be used in the present invention.

As used herein, "*chronic administration*," means near continuous administration of the drug for a period of at least four weeks, where *"near continuous administration"* is understood to include variations and interruptions in administration schedules routinely encountered in the field of medicine, but not interruptions of more than seven days.

The skin of a subject, for example, a mammalian subject, may be considered to be a membrane, wherein $m = (D \, K_m \, A/d) \, (Cs - Cb)t$, wherein $Cs$ = concentration of drug at the skin surface, $Cb$ = concentration of drug in systemic circulation, A = Area for absorption through skin thickness d, $K_m$ = Partition coefficient (SC and Sebum), m = Mass of drug diffusing in time t, and D = Drug's diffusion coefficient.

An exemplary embodiment provides compositions composed of a low molecular weight fatty acid having a long chain fatty acid or a long chain fatty acid lower alkyl ester, wherein the long chain is a $C_{13}$ to $C_{21}$ chain, optionally containing at least one cis-olefinic bond, which may be used as a transdermal delivery vehicle.

Another exemplary embodiment provides a chain length for the long chain of the fatty acid or fatty acid ester, excluding the carboxyl group carbon, between $C_{10}$ to $C_{19}$, including about $C_{17}$. In another exemplary embodiment, the long chain is a straight chain, which may optionally have only one cis-olefinic (C=C) bond. In another exemplary embodiment, the acid is lower alkyl esterified, with between about $C_1$ to about $C_4$ alkyl, including a $C_2$ alkyl. Thus myristeic acid is an example of a saturated acid of this embodiment, as are longer unsaturated acids such as oleic acid. In another exemplary embodiment, the low molecular weight fatty acid component is a $C_2$ to $C_4$ fatty acid, for example, propionic or butyric acid.

Interestingly, although oleic acid is well known as a skin irritant, in a 50:50 mixture with physostigmine, the composition is a non-irritant and penetration through the skin becomes very efficient. Ethyl oleate gives a better performance still and is optimal in combination with propionic acid.

Increasing the length of the long chain fatty acid may increase the disruption of tissue lipid layers, while decreasing the length is believed to reduce penetration of the composition across the dermis, including the phenserine compound which is contained within it. Therefore, a person of ordinary skill in the art may apply these factors, *inter alia*, when adapting the composition to a particular active ingredient composition and desired dosage level.

Exemplary ratios of the long chain fatty acid or its ester to the low molecular weight fatty acid are from about 40:60 to about 60:40 (vol.:vol.), or about 45:55 to about 55:45 (vol.:vol.), or about 50:50 (vol.:vol.), while the content of the active ingredient in this mixture may be from about 10 to about 50% (wt./vol.) of mixture, including from about 20 to 30% (wt./vol.) of mixture, and about 25% (wt./vol.).

The long chain fatty acid components oleic acid and ethyl oleate are acceptable for drug administration, but have never been used in a formulation of phenserine for a transdermal application. The use of an acidic component, such as the low molecular weight fatty acid, e.g., propionic acid, may be used to solvate and neutralise basic active agent e.g., phenserine free base.

The absorption through human skin of a phenserine compound may be used to determine the feasibility of transdermal delivery with a particular carrier or vehicle. For example, penetration of a phenserine compound across human epidermis may be measured *in vitro* using glass diffusion cells. Therefore, optimization of phenserine absorption is achieved by use of formulations disclosed herein and known in the art as penetration enhancers.

Clinical studies indicate that an oral dose of a phenserine compound of about 10 and/or 15 mg *bid* may produce a beneficial treatment for cognitive disorders, such as AD. This dosage does not cause overt signs of cholinergic over stimulation. While first pass metabolism of phenserine when administered orally does not appear to dramatically effect the necessary dosage, transdermal administration of phenserine is believed to be subject to reduced first pass metabolism. Therefore, the dosage of a phenserine compound may be reduced when administered transdermally.

When delivering a drug transdermally, if skin permeability becomes the limiting factor, bioavailability can be increased by using a larger surface area. Practical considerations limit the size of a patch for administration to humans to about 20 cm (20,000 μg / (24 hours x 400 cm$^2$) = permeability rate) which would require a permeability rate of at least 2.08 μg cm$^{-2}$ hr$^{-1}$ for transdermal delivery of this amount of phenserine in a 24 hour period. Higher permeability rates allow, *inter alia*, the patch size to be reduced.

To assay solubility, between 10-20 mg of phenserine is weighed into a 1 ml volumetric flask. An initial 1 ml volume of a chosen vehicle is added to the flask and the mixture agitated, for example, ultrasonically for at least 30 minutes. If dissolution does not occur at the end of this period, additional measured aliquots of the vehicle are added and the process repeated until complete dissolution occurs.

The partition coefficient ($K_m$) may be calculated for any vehicle using methods well known in the art, where the concentration of a phenserine compound in a tissue divided by the concentration in solution may be used to provide the $K_m$.

Saturated solutions of a phenserine compound may be prepared, for example, in isopropyl myristate, propylene glycol and propylene glycol/oleic acid (e.g., 50/50 vol./vol.) by adding an excess amount of the phenserine compound to a measured amount of vehicle. The solution is then agitated and allowed to stand, and the process of agitating and standing is repeated until dissolution of the compound is complete. The saturated solution is removed from any remaining settled phenserine compound prior to absorption studies.

Other formulations (wt./vol.) are prepared by weighing the phenserine compound and adding an appropriate amount of the vehicle. The mixture is then agitated until dissolution occurs.

Preparation of skin membranes: Human skin is obtained post mortem and may be stored at -20 °C until needed. When required the tissue is allowed to thaw at room temperature and the subcutaneous fat removed. Epidermal sheets are separated from full-thickness skin by immersing in water at 60 °C. for 45 seconds and gently peeling away the epidermis. Alternatively, skin may be obtained from another source, such as a mouse or rat.

Absorption studies are performed *in vitro* using glass diffusion cells, having epidermal membranes mounted in Franz type diffusion cells. For example, Franz type diffusion cells having a cross-sectional area of 2.54 cm$^2$. The cells may be housed in a water bath to maintain a desired temperature. The upper surface of the membrane is exposed to donor solution and the under surface of the membrane is bathed by continuously stirred receptor fluid. Receptor fluid may be a phosphate buffered saline solution, or any physiological solution.

Membrane integrity may be assayed using methods well known in the art prior to conducting a diffusion assay. For example, a membrane having a permeability coefficient greater than $1.5 \times 10^{-3}$ cm hr$^{-1}$ may be considered damaged and discarded. The compound, including vehicle and a phenserine compound are then applied to the donor chambers, and the movement of the phenserine compound across the membrane is followed by assaying the receptor solution for the presence of the phenserine compound. The receptor solution may be tested over any appropriate number of days, for example, over a period of seven days.

Results from absorption experiments are typically plotted as cumulative amount absorbed against time. The steady state penetration rate, or flux, J ($\mu$g/cm$^2$/hr), is typically calculated by linear regression analysis from the linear regions of an absorption plot. The permeability coefficient, Kp (cm/hr) is:

$$J = (Km \cdot D \cdot c)/s = Kp \cdot c$$

Where c is the penetrant concentration ($\mu$g/cm$^3$) in the donor chamber, Km is the partition coefficient of the penetrant between the vehicle and the skin. D is the diffusion coefficient of the penetrant in the epidermis and s is the membrane thickness.

A solid is defined as having a thermodynamic activity of unity, as a result, a saturated solution may also be considered to have an activity of unity. Therefore, the maximum penetration rate is expected to be achieved from saturated solutions of the drug. When penetration is less than 10% of applied dose, the conditions are a non-depleting donor source, which allows the assay to determine steady-state conditions.

Propylene glycol, isopropyl myristate, and propylene glycol/oleic acid (50/50 vol./vol.) are evaluated as vehicles for transdermal phenserine delivery. Formulations of phenserine in two component systems consisting of propionic acid/isopropyl myristate (50/50 vol./vol.) and propionic acid/oleic acid (50/50 vol./vol.) are evaluated against a formulation of 50% phenserine in propionic acid (as per WO 93/03767). The effects of changing the concentration of phenserine and the vehicle ratios may also be investigated.

Partitioning and solubility studies may be used to evaluate oleyl alcohol, ethyl oleate, squalene, dipropylene glycol monomethyl ether, and N-hydroxyethyl lactamide as aditional vehicles for transdermal phenserine delivery.

The time-course profiles for phenserine penetration across human epidermis from the formulations of the invention are conducted. Phenserine is preferably chemically stable in each formulation. Therefore, breakdown and degradation products, for example, by hydrolysis and subsequent oxidation, are assayed in each vehicle. The vehicles of the invention, including propionic acid and/or oleic acid, are assayed for the chemical stability of a phenserine compound. It is found that phenserine is chemically stable in the vehicles of the invention, such as propionic acid and/or oleic acid.

Propionic acid, as a co-solvent, with isopropyl myristate and/or oleic acid is found to increase the solubility and stability of phenserine. The two component based phenserine formulations, 25% in propionic acid/isopropyl myristate and 25% in propionic acid/oleic acid are found to achieve sufficiently high transdermal flux rates. Use of a saturated solution of phenserine may increase fluxes from the two-component systems and both two-component systems are found to be capable of delivering therapeutic amounts of phenserine via the skin from patches.

Although propionic acid is irritant, phenserine in propionic acid may form the propionate and thus reduce or eliminate irritancy in formulations containing high concentrations of phenserine. However, lower concentrations of a phenserine compound may result in sufficient free acid in the formulation to cause membrane damage.

To produce a non-damaging formulation containing 10% phenserine the ratio of propionic acid to oleic acid may be reduced from 50/50 to 10/90. Such a vehicle may be saturated with phenserine and is expected to be a non-irritant. The partition coefficient of phenserine from oleic acid is believed to be less favorable than from 50/50 propionic acid/oleic acid. Therefore, increasing the content of oleic acid may decrease the partitioning of phenserine into the epidermis.

Concentration and partitioning are critical determinants of transdermal flux. It follows that increases in $K_m$ and C will lead to higher flux rates. However, these two parameters are interdependent in that a vehicle with a high solubility for phenserine will be less likely to allow phenserine partitioning into skin and vice versa. It is believed that squalene, isopropyl myristate and ethyl oleate will have relatively low solubilities for phenserine but high $K_m$ values. Conversely it is believed that oleyl alcohol, dipropylene glycol monomethyl ether, N-hydroxyethyl lactamide and oleic acid have greater solubility for phenserine but lower $K_m$ values. A compromise between $K_m$ and c is desirable when optimizing vehicle formulations, and such a compromise is believed to be achieved with the vehicles of the invention, such as the two-component systems. In particular, ethyl oleate in combination with propionic acid is believed to provide a desirable vehicle for transdermal phenserine delivery.

Isopropyl myristate may enhance percutaneous absorption by increasing the partitioning of phenserine into skin. In addition, oleic acid and ethyl oleate may increase both the partitioning and diffusivity through the skin. Furthermore isopropyl myristate is believed to affect the stability of phenserine, therefore, use of the isopropyl myristate containing compositions of the invention may lead to slower production of break down products. Inclusion of oleic acid and ethyl oleate in transdermal formulations may allow the use of a smaller transdermal patch for a particular dosage.

A benefit of a transdermal dosage form includes improved subject compliance, due to the possibility of reduced administrations. For example, a transdermal patch of the invention may be formulated so as to provide one, two, three, four, five, six or seven days of medication. In an exemplary embodiment, the transdermal patch provides medication for about three days, before it is desirable to replace the patch. In another exemplary embodiment, the transdermal patch provides seven days of medication, before it is desirable to replace the patch. In another exemplary embodiment, the transdermal dosage form is a cream or lotion that may be applied to a subject. In addition, the transdermal dosage may be formulated with any desirable dosage of the phenserine compound. For example, the transdermal dosage may provide the equivalent dosage to oral administration of 5 mg *bid,* 10 mg *bid*, 15 mg *bid,* 20 mg *bid,* 25 mg *bid*, 30 mg *bid*, 35 mg *bid*, or 40 mg *bid.* As such, a transdermal patch(es) having the equivalent dosage to oral administration of 5 mg *bid* may be administered to a subject for a desired period of time, for example, four weeks, and then a patch or patches having the equivalent dosage to oral administration of 10 mg *bid* may be administered for a desired period of time. Optionally, additional or alternative dosage regimens may be administered, for example, a dosage equivalent to 15 mg *bid* may be administered.

Also included within the invention is a kit, which may contain a desired supply of patches, for example, a one month supply of transdermal patches. Optionally, a kit may be organized into a plurality of, e.g., three, differently identified (numbered, colored or the like) parts, wherein the contents of a first part are initially administered, followed by administration of the contents of a second part, which are then followed by administration of the contents of the third part.

Membranes useful in the construction of a transdermal patch are known in the art and include, but are not limited to, CoTran™ Membranes commercially available from 3M, such as the COTRAN™ 9701, 9702, 9705, 9706, 9715, 9716, 9726, and COTRAN™ 9728 membranes. Backing useful in the construction of a transdermal patch are known in the art and include, but are not limited to, backing material commercially available from 3M, such as COTRAN™ and SCOTCHPAK™ backings. Likewise, liners are well known in the art and may be obtained from a number of commercial sources. Optionally, a gelling agent may be optionally added at up to 20% by volume. Gelling agents, include, but are not limited to: crosslinked acrylic acid polymers, such as the "carbomer" family of polymers, e.g., carboxypolyalkylenes that may be obtained commercially under the tradename CARBOPOL™; hydrophilic polymers, such as polyethylene oxides, polyoxyethylene-polyoxypropylene copolymers and polyvinylalcohol; cellulosic polymers, such as hydroxypropyl cellulose, hydroxycthyl cellulose, hydroxypropyl methylcellulose, hydroxypropyl methylcellulose phthalate, and methyl cellulose; gums such as tragacanth and xanthan gum; sodium alginate; and gelatin.

Lotions or creams according to the invention, include preparations to be applied to the skin surface, and are typically liquid or semiliquid preparations in which solid particles, including the active agent, are present in a water or alcohol base. Lotions are usually suspensions of solids, and preferably, for the present purpose, comprise a liquid oily emulsion of the oil-in-water type. Lotions may be used for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like.

The effective dose for mammals may vary due to such factors as age, weight, activity level or condition of the subject being treated. Typically, an effective dosage of phenserine according to the present invention is between about 1 to about 800 milligrams when administrated by either oral or rectal dose from 1 to 3 times daily. This is about 0.002 to about 50 milligrams per kilogram of the subject's weight administered per day. Preferably about 10 to about 30 milligrams per day are administered for an adult human. A transdermal delivery vehicle of the invention may be applied to a subject for any desirable period of time, for example, daily, weekly, and biweekly.

The invention also relates to a method of reducing symptoms in a subject believed to be suffering from or susceptible to a symptomatic state susceptible to treatment by a combination of phenserine and (+)-phenserine, POSIPHEN™, or pharmaceutically acceptable salts or esters thereof, the method comprising: transdermally co-administering to the subject a first dose of phenserine or a pharmaceutically acceptable salt or esters thereof, together with a fixed dose of POSIPHEN™ for a first period of time; thereafter, transdermally co-administering to the subject a second dose of phenserine or a pharmaceutically acceptable salt or ester thereof, said second dose greater than said first dose, again together with the fixed dose of POSIPHEN™ for a second period of time; and transdermally co-administering to the subject a maintenance dose of phenserine or a pharmaceutically acceptable salt or ester thereof, said maintenance dose greater than said second dose, together with the fixed dose of POSIPHEN™ for a period of time necessary to reduce or prevent symptoms in the subject. The time periods may be between about one week and about 6 weeks and may be adjusted to reduce symptoms of cholinergic over stimulation.

The suitability of the compositions of the invention for such transdermal delivery of active ingredients will now be illustrated by way of the following illustrative Examples, which are provided to aid a man skilled in the art to better comprehend their advantages. Other suitable compositions falling within the scope of the invention will occur to a skilled man in the light of these Examples.

Example I

Propionic acid (500 ml, commercially available from Sigma) is slowly added to 500 ml of liquid ethyl oleate (commercially available from Aldrich) to form a propionic acid/ethyl oleate (50/50, vol./vol.) solution.

The propionic acid/ethyl oleate solution is saturated with phenserine, by adding about 50 g of phenserine to the solution and dissolving the phenserine by repeated sonication.

The saturated solution is prepared as a gel for ease of handling, for example, by the addition of Carbopol polymers to produce a gel.

The gelled formulation is then metered onto a piece of backing film (such as, 1006 Scotchpak polyester film laminate, available from 3M) in the amount of about 300 mg per square centimeter and is covered with a tri-laminate consisting of three films (Adhesives Research), namely, a porous membrane/acrylic adhesive/release liner with the porous membrane side facing the gel. The tri-laminate and the backing film are heat-sealed, for example, using an impulse heat sealer, into a circular configuration to form a reservoir. The saturated solution is applied in a reservoir style transdermal patch. For example, the patch may be produced using a CoTran™ 9701 membrane heat sealed to a backing to form a reservoir between the membrane and the backing.

Example II

Phenserine is prepared as a lotion or cream which contains about 10 mg of phenserine per gram of cream. The cream base comprises purified water, propionic acid, propylene glycol, ethyl oleate, and benzoic acid as a preservative. Optionally, the lotion is measured out into individual containers, such as a foil pouch. The subject or an assistant may then apply the lotion, thereby treating a cognitive disorder.

Example III

An adsorption studies is carried out and the experimental variables are optimized to achieve maximum adsorption of phenserine and/or (+)-phenserine on a polymer. The influence of an electrolyte on adsorption is determined using sodium chloride in different concentrations. (+)- and/or (-)-phenserine adsorbed to the polymer is prepared by placing a known weight of the polymer (approximately 200-225 $\mu$m) in contact with a saturated solution of the drug (in 95% v/v ethanol, or Tween 80:ethanol 3:1 v/v), optionally containing a physiologically acceptable salt, e.g., about 0.1% w/v sodium chloride, for a period of about 5 hours. The polymer is then removed by filtration and dried under vacuum at low temperature.

The equivalent of 180 mg of phenserine adsorbed polymer is dissolved in about 10 ml 40%w/w PEG-400. The mixture is stirred at a low speed and the polymeric solution is cast into foil cups of area 20 cm$^2$ and dried at room temperature. An adhesive layer is formed by adding about 5 ml polyisobutylene, (50%w/v in a solvent), to the dried film surface and the solvent is evaporated to form an adhesive layer

*In vitro* diffusion studies using a modified Keshary-Chein diffusion cell, epithelium of the fresh human cadaver, and distilled water as the receptor medium are carried out to determine the diffusion rate. An agitation speed of approximately 50 rpm and a temperature of about 37 $^\circ$C maintained is used. Samples are withdrawn from the receptor compartment at hourly intervals and the quantity of the drug(s) are determined. In one embodiment, the *desired in vitro* flux rate for (-)-phenserine is approximately 62.5 $\mu$g/hr/cm$^2$. As will be recognized by a person of ordinary skill, in light of the description herein, the flux rate for (+)-phenserine may be significantly higher. Since the two enantiomers are not expected to exhibit a difference in sorption or release, a transdermal patch having the proper ratio of (-)-phenserine:(+)-phenserine may be prepared.

The rate of drug release is presumed to follow standard parameters, *see,* for example, A.T. Florence. and D. Attwood., Physicochemical principles of Pharmacy, 119 (3rd ed. Macmillan Press Ltd, London, 1998); and Narasimha *et al*. (2001) Formulation And Evaluation Of Controlled Release Transdermal Patches Of Theophylline - Salbutamol Sulphate, *The Internet Journal of Pharmacology* Volume 1, Number 1.

The rate of drug release is assayed *in vivo* using male rats. A small patch of fur is removed by shaving, without compromising the integrity of the skin, from the back of the male rats. Catheters, filled with heparinized saline, are tied into the right femoral vein and artery of anesthetized male rats, which are restrained in a plaster cast and allowed to recover from anesthesia in a temperature-controlled enclosure. Plasma samples are withdrawn to quantify the level of AChE activity in the untreated state. After surgery, a transdermal patch of the appropriate size is applied to the exposed skin and plasma samples are removed at intervals between 2 minutes and 8 to 48 hours. If cholinergic over-stimulation is anticipated, hexamethonium bromide (5 mg/kg. i. p.) followed by atropine methylbromide (4mg/kg, s.c.) may be administered prior to application of the transdermal patch (*see,* U.S. Patent 5,998,460). These quaternary nicotinic and muscarinic antagonists do not enter brain and inhibit peripheral cholinergic over-stimulation, which may be deleterious to the animal. The plasma samples are immediately frozen at -70 °C and AChE inhibition is measured as previously described, with necessary modifications required for quantitation from rat plasma (*see,* U.S. Patent 5,998,460).

While this invention has been described in certain embodiments, the present invention can be further modified within the spirit and scope of this disclosure and claims.

## Claims

1. Use of a phenserine compound for the manufacture of a transdermal medicament for reducing beta-amyloid protein accumulation, wherein the transdermal medicament comprises a long chain fatty acid or a long chain fatty acid lower alkyl ester, wherein the long chain is a $C_2$ to $C_{21}$ chain, and the phenserine compound in the transdermal medicament penetrates the stratum corneum and crosses the blood brain barrier at a rate sufficient to inhibit translation of beta-amyloid precursor protein.

2. The use of claim 1, wherein the transdermal medicament comprises a long chain fatty acid or its ester and a low molecular weight fatty acid in a ratio of about 40:60 to about 60:40 (vol.:vol.).

3. The use of claim 1 or claim 2, wherein the long chain comprises at least one cis-olefinic bond.

4. The use of any one of claims 1-3, wherein the transdermal medicament comprises propionic acid, isopropyl myristate and/or oleic acid.

5. The use of claim 4, wherein the transdermal medicament comprises isopropyl myristate.

6. The use of claim 4, wherein the transdermal medicament comprises propylene glycol.

7. The use of claim 4, wherein the transdermal medicament comprises a propionic acid/ethyl oleate (50/50, vol./vol.) solution.

8. The use of claim 4, wherein the transdermal medicament comprises a propionic acid/isopropyl myristate (50/50 vol./vol.) solution.

9. The use of claim 4, wherein the transdermal medicament comprises a propylene glycol/oleic acid (50/50 vol./vol.) solution.

10. The use of claim 4, wherein the transdermal medicament comprises a propionic acid/oleic acid (50/50 vol./vol.) solution.

11. The use of any one of claims 1-10, wherein the transdermal medicament comprises a propylene glycol and oleic acid.

12. The use of any one of claims 1-10, wherein the transdermal medicament is administered weekly.

13. The use of any one of claims 1-10, wherein the transdermal medicament is applied in a plurality of dosages.

14. A kit for administering a phenserine compound to a subject, said kit comprising in separate containers, in one or more packages, an effective amount of a phenserine compound or a pharmaceutically acceptable salt or ester thereof in a transdermal formulation, wherein the phenserine compound penetrates the stratum corneum and crosses the blood brain barrier of the subject at a rate sufficient to inhibit translation of beta-amyloid precursor protein upon chronic administration of the phenserine compound.

15. A multiphasic beta amyloid precursor protein reducing kit comprising at least two phases, wherein the two phases are administered transdermally:

a first phase of separate first dosage units, each said first dosage unit comprising transdermal formulation comprising phenserine or a pharmaceutically acceptable salt or ester thereof, wherein the transdermal formulation has a flux rate sufficient to administer the phenserine at a dosage equivalent approximately equal to an oral dosage of phenserine of about 20 mg/day and a pharmaceutically acceptable carrier; and
a second phase of separate second dosage units, each said second dosage unit comprising transdermal formulation comprising phenserine or a pharmaceutically acceptable salt or ester thereof, wherein the transdermal formulation has a flux rate sufficient to administer phenserine at a dosage equivalent approximately equal to an oral dosage of phenserine of about 30 mg/day and a pharmaceutically acceptable carrier.

16. The kit of claim 15, further comprising a third phase of separate second dosage unit comprising transdermal formulation comprising phenserine or a pharmaceutically acceptable salt or ester thereof, wherein the transdermal formulation has a flux rate sufficient to administer phenserine at a dosage equivalent approximately equal to an oral dosage of phenserine of about 10 mg/day and a pharmaceutically acceptable carrier.

17. The kit of claim 15, further comprising a third phase of separate second dosage unit comprising transdermal formulation comprising phenserine or a pharmaceutically acceptable salt or ester thereof, wherein the transdermal formulation has a flux rate sufficient to administer phenserine at a dosage equivalent approximately equal to an oral dosage of phenserine of about 40 mg/day and a pharmaceutically acceptable carrier.

18. The kit of claim 15, further comprising a third phase of separate second dosage unit comprising transdermal formulation comprising phenserine or a pharmaceutically acceptable salt or ester thereof, wherein the transdermal formulation has a flux rate sufficient to administer phenserine at a dosage equivalent approximately equal to an oral dosage of phenserine of about 60 mg/day and a pharmaceutically acceptable carrier.

19. A transdermal pharmaceutical composition for impeding translation of beta amyloid precursor protein in a subject, said pharmaceutical composition comprising:

a pharmaceutically acceptable vehicle for carrying an active ingredient and an active ingredient comprising a phenserine compound, wherein the phenserine compound is present in the pharmaceutically acceptable vehicle in an amount sufficient to interfere with translation of beta amyloid precursor protein in the subject after application of the transdermal pharmaceutical composition to the subject's skin and delivery of the phenserine compound to the subject.

20. The transdermal pharmaceutical composition of claim 19, comprising a low molecular weight fatty acid and a long chain fatty acid or a long chain fatty acid lower alkyl ester, wherein the long chain, excluding a carboxyl carbon, is between 2 to 21 carbons, and a phenserine compound or pharmaceutically acceptable salts or esters thereof.

21. The transdermal pharmaceutical composition of claim 19, comprising a $C_2$ to $C_4$ fatty acid in combination with a $C_2$ to $C_4$ alkyl ester of oleic acid as the vehicle for the phenserine compound.

22. The transdermal pharmaceutical composition of claim 21, wherein the fatty acid is propionic acid and the oleic acid is ethyl oleate.

23. The transdermal pharmaceutical composition of any one of claims 19-22, wherein the long chain of the long chain fatty acid contains at least one cis-olefinic bond.

24. The transdermal pharmaceutical composition of any one of claims 19-23, wherein the low molecular weight fatty acid is propionic acid.

25. The transdermal pharmaceutical composition of claim 19, wherein the long chain of the long chain fatty acid or fatty acid ester is a $C_{15}$ to $C_{19}$ chain.

26. The transdermal pharmaceutical composition of claim 19, wherein the long chain fatty acid is a straight chain.

27. The transdermal pharmaceutical composition of claim 19, wherein the long chain fatty acid comprises only one cis-olefinic group.

28. The transdermal pharmaceutical composition of claim 27, wherein the long chain fatty acid is oleic acid.

29. The transdermal pharmaceutical composition of claim 19, wherein the long chain fatty acid is ester is formed with a $C_1$ to $C_4$ alkyl.

30. The transdermal pharmaceutical composition of claim 29, wherein the long chain fatty acid ester is ethyl oleate.

31. The transdermal pharmaceutical composition of claim 19, wherein the long chain fatty acid ester is isopropyl myristate.

32. The transdermal pharmaceutical composition of claim 20, wherein the ratio of the long chain fatty acid or long chain fatty acid ester to the low molecular weight fatty acid is from 40:60 to 60:40 Vol./Vol.

33. The transdermal pharmaceutical composition of claim 32, wherein the ratio of the long chain fatty acid or long chain fatty acid lower alkyl ester to the low molecular weight fatty acid is about 50:50 Vol./Vol.

34. The transdermal pharmaceutical composition of claim 20, comprising 10 to 50% wt./vol. of the phenserine compound.

**35.** The transdermal pharmaceutical composition of any one of claims 19-34, wherein the phenserine compound is (3aR)-1,3a,8-trimethyl-1,2,3,3a,8,8a-hexahydropyrrolo[2,3-b]indol-5-yl phenylcarbamate.

**36.** The transdermal pharmaceutical composition of any one of claims 19-34, wherein the phenserine compound is (3aR)-1,3a,8-trimethyl-1,2,3,3a,8,8a-hexahydropyrrolo[2,3-b]indol-5-yl phenylcarbamate tartrate.

**37.** The transdermal pharmaceutical composition of any one of claims 19-34, wherein the phenserine compound is (3aS)-1,3a,8-trimethyl-1,2,3,3a,8,8a-hexahydropyrrolo[2,3-b]indol-5-yl phenylcarbamate.

**38.** The transdermal pharmaceutical composition of any one of claims 19-37, comprising a delivery tape or patch.

**39.** The transdermal pharmaceutical composition of any one of claims 19-38 for use in the treatment of a cognitive disorder associated with beta-amyloid protein accumulation.

**40.** The transdermal pharmaceutical composition of any one of claims 19-38 for use in the treatment of Alzheimer's disease.

**41.** Use of the transdermal pharmaceutical composition of any one of claims 19-38 in the manufacture of a medicament for treatment or prevention of a cognitive disorder, wherein the medicament comprises at least two transdermal formulations comprising a combination of (-)-phenserine and (+)-phenserine or pharmaceutically acceptable salts or esters thereof, and wherein the medicament comprises:

a first transdermal formulation having a first dose of phenserine or a pharmaceutically acceptable salt or esters thereof, together with a fixed dose of (+)-phenserine; and
a second transdermal formulation having a second dose of phenserine or a pharmaceutically acceptable salt or ester thereof, said second dose being greater than said first dose, again together with the fixed dose of (+)-phenserine.

**42.** The use of claim 41, further comprising a third transdermal formulation having a third dose of phenserine or a pharmaceutically acceptable salt or ester thereof, said third dose being greater than said second dose, together with the fixed dose of (+)-phenserine.

**43.** The use of claim 41 or claim 42, wherein the first dose of phenserine is equivalent to an oral dosage of phenserine of about 10 mg twice a day.

**44.** The use of claim 41, claim 42, or claim 43, wherein the second dose of phenserine is equivalent to an oral dosage of phenserine of about 15 mg twice a day.

**45.** The use of claim 41, claim 42, or claim 43, wherein the second dose of phenserine is equivalent to an oral dosage of phenserine of about 20 mg twice a day.

**46.** The use of claim 43, wherein the third dose of phenserine is equivalent to an oral dosage of phenserine of about 60 mg twice a day.

FIG. 1

Reservoir

☐ Backing

■ Drug

▨ Membrane

☐ Adhesive

▨ Liner

FIG. 2

Matrix

▨ Backing

☐ Adhesive

■ Drug

▨ Liner

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94/12176 A (THE UNITED STATES OF AMERICA) 9 June 1994 (1994-06-09) | 1-46 | A61K9/70 A61K31/40 |
| Y | * page 1, paragraph 2 * <br> * page 25 - page 28 * | 1-46 | |
| X | SPANGLER E L et al. "Transdermal application of the actehylcholineesterase inhibitor phenserine attentuates ..." Soc. for Neuroscience Abstract Viewer and Iterinary Planner 2002 32nd Ann Meeting of the Soc. for Neuroscience, Orlando, Florida, USA, Nov 2002 Abst.no. 889.5 XP009053121 * abstract * | 1-46 | |
| X | WO 2004/034963 A (EISAI CO., LTD; IENI, JOHN; PRATT, RAYMOND) 29 April 2004 (2004-04-29) * page 24, line 34 - page 25, line 24; claims 4,15 * | 19-40 | |
| Y,D | WO 93/03767 A (THE SECRETARY OF STATE FOR DEFENCE IN HER BRITANNI) 4 March 1993 (1993-03-04) * page 3 - page 4 * | 1-46 | **TECHNICAL FIELDS SEARCHED (Int.Cl.7)** <br> A61K |
| Y | US 5 364 629 A (KOCHINKE ET AL) 15 November 1994 (1994-11-15) * claim 1 * | 1-46 | |

The present search report has been drawn up for all claims

| Place of search | Date of completion of the search | Examiner |
|---|---|---|
| Munich | 31 August 2005 | Bendl, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 07 6267

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2005

| Patent document cited in search report | | Publication date | Patent family member(s) | | Publication date |
|---|---|---|---|---|---|
| WO 9412176 | A | 09-06-1994 | US | 5409948 A | 25-04-1995 |
| | | | AT | 213411 T | 15-03-2002 |
| | | | AU | 693428 B2 | 02-07-1998 |
| | | | AU | 5729094 A | 22-06-1994 |
| | | | CA | 2149924 A1 | 09-06-1994 |
| | | | DE | 69331605 D1 | 28-03-2002 |
| | | | DE | 69331605 T2 | 31-10-2002 |
| | | | DK | 668763 T3 | 03-06-2002 |
| | | | EP | 0668763 A1 | 30-08-1995 |
| | | | ES | 2173111 T3 | 16-10-2002 |
| | | | JP | 8505130 T | 04-06-1996 |
| | | | PT | 668763 T | 30-08-2002 |
| | | | WO | 9412176 A1 | 09-06-1994 |
| WO 2004034963 | A | 29-04-2004 | AU | 2003298514 A1 | 04-05-2004 |
| | | | WO | 2004034963 A2 | 29-04-2004 |
| WO 9303767 | A | 04-03-1993 | AU | 660487 B2 | 29-06-1995 |
| | | | AU | 2460292 A | 16-03-1993 |
| | | | CA | 2115831 A1 | 04-03-1993 |
| | | | EP | 0599952 A1 | 08-06-1994 |
| | | | WO | 9303767 A1 | 04-03-1993 |
| | | | GB | 2274778 A ,B | 10-08-1994 |
| | | | IL | 102901 A | 29-02-2000 |
| | | | RU | 2117490 C1 | 20-08-1998 |
| | | | US | 5612056 A | 18-03-1997 |
| US 5364629 | A | 15-11-1994 | AU | 647123 B2 | 17-03-1994 |
| | | | AU | 7736991 A | 17-12-1992 |
| | | | EP | 0523150 A1 | 20-01-1993 |
| | | | WO | 9115176 A1 | 17-10-1991 |
| | | | JP | 5506026 T | 02-09-1993 |

EPO FORM P0459

For more details about this annex : see Official Journal of the European Patent Office, No. 12/82